# EUROPEAN PATENT SPECIFICATION

(11) **EP 0 671 905 B1**
(45) Date of publication and mention of the grant of the patent: **28.08.2002**
(21) Application number: 94929959.8
(22) Date of filing: 29.09.1994
(51) Int. Cl.: A61K 9/127, A61K 38/19

(54) **STABLE PROTEIN: PHOSPHOLIPID COMPOSITIONS AND METHODS**
STABILE PROTEIN-PHOSPHOLIPID ZUSAMMENSETZUNGEN UND VERFAHREN
COMPOSITIONS STABLES PROTEINES-PHOSPHOLIPIDES ET PROCEDES

(30) Priority: 06.10.1993 US 132413
(43) Date of publication of application: 20.09.1995
(73) Proprietor: Amgen Inc., Thousand Oaks, CA 91320-1799 (US)
(72) Inventor: COLLINS, David, Thousand Oaks, CA 91360 (US); CHA, Younsik, Thousand Oaks, CA 91360 (US)
(74) Representative: Brown, John David
(86) International application number: US9411081
(87) International publication number: WO95009611

(56) References cited:
- WO-A-93/18749
- DATABASE WPI Week 9436 Derwent Publications Ltd., London, GB; AN 94-293949 & WO,A,94 18948 (GREEN CROSS CORP.) , 1 September 1994
- FEBS LETTERS, vol. 262,no. 1, 1990 pages 20-24, O.B. PTITSYN ET AL. 'Evidence for a molten globule state as a general intermediate in protein folding.' cited in the application
- NATURE, vol. 354, 1991 pages 408-410, F.G. VAN DER GOOT ET AL 'a 'molten globule' membrane insertion intermediate of the pore-forming domain of colicin a.' cited in the application
- ACS SYMPOSIUM SERIES, vol. 526, 1993 pages 156-163, P.M. HOROWITZ 'Kinetic control of protein folding by detergent micelles, liposomes and chaperonins.'

## Description

### BACKGROUND OF THE INVENTION

The present invention relates to stabilizing the secondary and tertiary structure of proteins capable of transitioning into the molten globular state. More particularly, this invention relates to G-CSF:phospholipid compositions having increased stability, and capable of use in G-CSF formulations and in novel G-CSF delivery vehicles.

Several proteins have been shown to transition into a molten globular state (MGS). Van der Goot, F.G., *Nature* 354, 408-410 (1991), Proteins in the molten globular state exhibit secondary structure which is comparable to the native protein yet they lack rigid tertiary structure. Pitsyn et al., *FEBS Letters* 262:1, 20-24 (1990). In some cases, transition into this state is accompanied by exposure of previously hidden hydrophobic regions of the protein. By exposing critical hydrophobic residues, the MGS may be an intermediate in the aggregation and precipitation of proteins. The MGS conformation can be detected by comparing the circular dichroism in the far UV region with the spectra of aromatic side chains (near UV circular dichroism and fluorescence). The molten globular state exhibits aromatic group spectral changes in the absence of far UV circular dichroism changes, Bychkova et al., *FEES Letters* 238: 231-234 (1988), and may be involved in membrane penetration by some proteins Bychkova et al., *FEBS Letters* 238: 231-234 (1988); Van der Goot, F.G., *Nature* 354, 408-410 (1991).

Two proteins known to transition into the MGS prior to aggregation are granulocyte colony stimulating factor (G-CSF) and granulocyte macrophage colony stimulating factor (GM-CSF). While these two proteins may be stabilized under certain defined conditions, there exists a need to extend the shelf life of these materials by stabilizing the secondary and tertiary structure of the proteins.

Human recombinant G-CSF selectively stimulates neutrophils, a type of white blood cell used for fighting infection. Currently, Filgrastim, a recombinant G-CSF, is available for therapeutic use. The structure of G-CSF under various conditions has been extensively studied; Lu et al., *J. Biol. Chem*. Vol. 267, 8770-8777 (1992). Because of its hydrophobic characteristics, G-CSF is difficult to formulate for extended shelf life. Formulations of certain hydrophobic proteins lose activity due to formation of dimer and higher order aggregates (macro range) during long-term storage. Other chemical changes, such as deamidation and oxidation may also occur upon storage. In addition, the G-CSF formulator must protect against denaturation and, in particular, look to maintain the stability of the secondary and tertiary structure of the protein.

Human GM-CSF is a 22-kDa glycoprotein required continuously for the *in vitro* proliferation of macrophage and granulocytic progenitor cells. It also controls the irreversible commitment of these progenitors to form granulocytes and macrophages. Other biological activities may include regulation of the functional activity of mature cell types; Gough et al., *Nature*, 309, 763-767 (1984), and increasing chemotaxis towards recognized chemoattractants; Williams et al., *Hematology*, 4th ed. (1990). GM-CSF also stimulates the production of monocytes, and thus may be useful in the treatment of monocytic disorders, such as monocytopenia.

Human GM-CSF can be obtained and purified from a number of sources. Procedures for the production of recombinant human GM-CSF have been previously described by Burgess et al., *Blood,* 69:1, 43-51 (1987). U.S. Patent 5,047,504 (Boone) has enabled the production of commercial scale quantities of GM-CSF in non-glycosylated form as a product of procaryotic host cell expression.

One way which has been tried in the past to work with proteins such as G-CSF and GM-CSF is the use of liposomes. Liposomes are completely closed lipid bilayer membranes formed by water-insoluble polar lipids, particularly phospholipids. Liposome vesicles may have a single membrane bilayer (unilamellar) or may have multiple membrane bilayers (multilamellar). The bilayer is composed of two lipid monolayers having a hydrophilic (polar) "head" region and a hydrophobic (nonpolar) "tail" region wherein the hydrophobic tails orient toward the center of the bilayer, whereas the hydrophilic heads orient toward the aqueous phase. The stability, rigidity, and permeability of liposomes can be altered by changes in the phospholipid composition, by changes in temperature, by inclusion of a sterol or by incorporation of a charged amphiphile. The basic structure of liposomes may be made by a variety of techniques known in the art.

In the process of their formation liposomes can entrap water solutes in the aqueous channels and release them at variable rates. Upon the discovery that liposomes can introduce enzymes into cells and alter their metabolism (Gregoriadis, *New Engl. J. Med*. 295, 704-710, 765-770 (1976)), liposomes were heralded as the answer to the quest for targeted drug delivery. As a result, there is a great deal of developmental research in the pharmaceutical industry involving use of liposomes as slow release depots for drugs, vitamins and proteins sequestered within the hydrophobic layers or hydrophobic core of the liposome.

Successful use of liposomes as drug-carriers has been limited because the researchers attempting such use have encountered several problems. For example, liposomes are known to act as powerful immunological adjuncts to entrapped antigens and caution must be exercised when enzymes or other proteins of xenogenic origin are entrapped in the liposomes. Also, the rate of diffusion of the drug is difficult to control. This is a function of the inherent instability of the liposomes and the presence of certain blood components which accelerate diffusion of certain drugs. In addition, by their nature, some substances are poorly entrapped in liposomes and therefore diffuse rapidly in circulation. Finally, there has been a problem targeting any cells or organ other than the liver or spleen. An excellent review of liposomes, substances which have been incorporated into liposomes, and the problems associated with use of liposomes as drug carriers is "Liposomes" by Gregory Gregoriaidis, found in *Drug Carriers in Biology and Medicine,* Chapter 14, 287-341 (Academic Press, N.Y., 1979).

While much has been reported concerning attempts to use liposomes as drug carriers, little has been disclosed concerning the use of liposomes for purposes of increasing shelf life of therapeutic peptides or proteins by stabilizing the structure of the peptide and/or protein. In PCT/US90/05163, entitled, *"Therapeutic Peptides and Proteins",* Hostetler, et al. disclose use of empty liposomes as a pharmaceutically acceptable diluent to solubilize polypeptides and/or proteins in order to prevent accumulation of the polypeptides and/or proteins at an air/water interface, and to prevent adsorption of the polypeptides and/or proteins to container surfaces. Hostetler et al. disclose that negatively charged phospholipid may be added up to about 50 mole percent, and that Phosphatidychloine, a neutral phospholipid, is the preferred lipsome. Hostetler et al. do not disclose a diluent shown to stabilise the structure of a polypeptide and/or protein.

In PCT/US91/07694, entitled, "*Preparation and Characterization of Liposomal Formulatios of Tumor Necrosis Factor*", Hung et al., a lipophilic modified tumor necrosis factor (TNF) molecule in association with the surface or encapsulated within a lipsome is described. The liposomal lipophilic TNF molecules are reported to have enhanced *in vivo* stability. Stability referred to a decrease or decreased tendency of the TNF-liposome to leak TNF into the system *in vivo*. The preferred liposome were neutral lipids. Hung et al. do not disclose a TNF composition wherein the excipients have a stabilising effect on the structure of the protein.

WO-A-93/18749 discloses a new class of lipsomes which exhibit an increased liver uptake. This enhanced uptake is allegedly due to a negatively charged carrier substance. The lipsomes can be used to produce pharmaceutical compositions wherein certain water-insoluble active substances are encapsulated within the liposomes . WO-A-94/18948 discloses encapsulation of proteins into liposomes.

Nothing can be drawn from the literature concerning contacting a protein, e.g. G-CSF, with a negatively charged lipid vesicle thereby directly stabilizing the protein against thermally-induced aggregation, denaturation, loss of activity, and unfolding of the secondary structure. The exists for such compositions which provide the benefit of being useful in formulation procedures requiring high temperatures as well as being used as novel delivery vehicles (e.g. oral administration of pegylated G-CSF). The present invention provides such compositions.

### SUMMARY OF THE INVENTION

According to the present invention there is a process for preparing a pharmaceutical composition in which a protein is stabilised against unfolding of the secondary structure of the protein which process comprises contacting the protein, which protein is capable of transitioning into the molten globular state with a negatively charged liposome vesicle, wherein the liposome-protein complex directly stabilises against unfolding of the secondary structure of the protein and obtaining a liposome-protein pharmaceutical composition.

In particular, the invention is directed to preparing stable G-CSF:phospholipid compositions. Surprisingly, the preferred G-CSF compositions can be cycled several times between 10-95°C with full recovery of protein secondary structure upon cooling. These compositions have the dual benefit of being useful for formulation procedures requiring high temperatures, as well as use in novel G-CSF delivery vehicles.

In a preferred embodiment, the negatively charged liposome is selected from dioleoylphosphatidylglycerol (DOPG), dimyristoylphosphatidylglycerol (DMPG), dipalmitoylphosphatidylglycerol (DPPG), eggphosphatidylglycerol, dioleoylphosphatidylethanolamine (DOPE), eggphosphatidylethanolamine, dioleoylphosphatidic acid (DOPA), dimyristoylphosphatidic acid (DMPA), dipalmitoylphosphatidic acid (DPPA), dioleoylphosphatidylserine (DOPS), dimyristoylphosphatidylserine (DMPS), dipalmitoylphosphatidylserine (DPPS), eggphosphatidylserine, lysophosphatidylglycerol, lysophosphatidylethanolamine, lysophosphatidylserine. DOPG, a negatively charged, unsaturated phospholipid is especially preferred. The invention further preferably comprises a pH maintained in the range of from 3.0 to 7.5, and a lipid:protein ratio of at least 10:1.

Additional elements that provide preferred embodiments of the invention include use of chemically modified proteins in the protein:phospholipid complex as well as use of one or more of the following: an isotonicity adjusting agent; a buffering agent; and a pH adjusting agent. As would be understood by a person having knowledge of the art, the invention encompasses stable protein:phospholipid compositions having various combinations of these additional elements, so long as they have been prepared by the process of the invention.

### BRIEF DESCRIPTION OF THE DRAWINGS

FIGURE 1 depicts the fluorescence emission spectrum of rhG-CSF in the presence (curve 1) and absence (curve 2) of DOPG vesicles. The concentration of the rhG-CSF was 0.2 mg/ml. The DOPG:rhG-CSF ratio (curve 1) was 100:1 (mole:mole).

FIGURE 2 shows the effect of increasing lipid:protein ratio on the rhG-CSF fluorescence. F₀ is the initial fluorescence (no lipid) and F refers to the fluorescence after addition of lipid to achieve the indicated molar ratio of lipid:rhG-CSF. Figure 2(a) shows F/F₀ (n) and maximum emission wavelengths (Δ) for mixtures of DOPG:rhG-CSF. Figure 2(b) shows F/F₀ (n) and maximum emission wavelengths (Δ) for mixtures of DOPC:rhG-CSF.

FIGURE 3 shows Stern-Volmer plots of the quenching of rhG-CSF fluorescence by KI in the absence (·) and presence (o) of DOPG vesicles. Quenching experiments were performed by adding aliquots of KI to rhG-CSF (0.2 mg/ml) and DOPG:rhG-CSF (100:1 molar).

FIGURE 4 depicts quenching of rhG-CSF tryptophan fluorescence upon addition of pyrene decanoic acid. The emission wavelength was 327 nm. The DOPG:rhG-CSF ratio was 100:1 (molar).

FIGURE 5 is a graph showing a comparison of F intensity changes for rhG-CSF in the absence and presence of various lipids. In each case, the lipid:protein ratio was 100:1 (molar).

FIGURE 6 is a graph showing a comparison of shifts in emission maximums for rhG-CSF in the absence and presence of various lipids. In each case, the lipid:protein ratio was 100:1 (molar).

FIGURE 7 shows the effect of DMPC (curve 2), DMPG (curve 3), and DMPA (curve 4) on the CD of rhG-CSF (curve 1). In each case, the lipid:protein ratio was 50:1 (molar) in water, pH 6.0.

FIGURE 8 shows the effect of increasing temperature on the CD of rhG-CSF (curve 1) or DOPG:rhG-CSF (140:1 molar) (curve 2). The rhG-CSF concentration was 80 µg/ml in water, pH 6.0. The temperature was scanned from 10-90°C at a rate of 100°C/hour.

FIGURE 9 shows differential scanning calorimetry thermograms for rhG-CSF (curve 1) and DOPG:rhG-CSF (45:1 molar) (curve 2). The concentration of rhG-CSF in the samples was 1 mg/ml (pH 7,0 in water). The scan rate was 90°C/hour.

FIGURE 10 shows the effect of temperature cycling on the CD of rhG-CSF (curve 1) and DOPG:rhG-CSF (140:1 molar) (curve 2). The samples were rapidly heated to 95°C and cooled to 10°C as indicated by the arrows. The rhG-CSF concentration in the samples was 80 µg/ml, pH 6.0.

FIGURE 11 shows the effect of temperature cycling on the CD of rhG-CSF (curve 1) and DMPG:rhG-CSF (150:1 molar) (curve 2). The samples were heated to 95°C and cooled to 10°C. The rhG-CSF concentration in the samples was 80 µg/ml, pH 6.0.

FIGURE 12 shows the effect of temperature cycling on the CD of rhG-CSF (curve 1) and DPPG:rhG-CSF (150:1 molar) (curve 2). The samples were heated to 95°C and cooled to 10°C. The rhG-CSF concentration in the samples was 80 µg/ml, pH 6.0.

FIGURE 13 is a graph showing the ability of various lipids to stabilize rhG-CSF during freeze-drying. The lipid:protein ratio was 100:1 in each case. Stability was based upon retention of *in vitro* activity in the bone marrow assay. rhG-CSF alone does not survive the freeze-drying process so the control used is untreated rhG-CSF in the absence of lipid.

FIGURE 14 shows the effects of various lipids on the *in vivo* activity of rhG-CSF. Activity (WBC count) was measured after subcutaneous injection of hamsters. The rhG-CSF dose was 100 µg/kg with a 100:1 lipid:protein ratio.

FIGURE 15 shows the effects of various lipids on the *in vivo* activity of rhG-CSF. Activity (WBC count) was measured after subcutaneous injection of hamsters. The rhG-CSF dose was 100 µg/kg with a 50:1 lipid:protein ratio.

FIGURE 16 is a graph showing a comparison of F intensity changes for CHO-G-CSF in the absence and presence of DOPG at varying pH's. In each case, the lipid:protein ratio was 100:1 (molar).

FIGURE 17 is a graph showing a comparison of shifts in emission maximums for CHO-G-CSF in the absence and presence of DOPG at varying pH's. In each case, the lipid:protein ratio was 100:1 (molar).

FIGURE 18 shows the effect of temperature cycling on the CD of PEG-G-CSF (―) and DMPG:PEG-G-CSF (17:1 molar) (----). The samples were heated to 90°C and cooled to 10°C.

FIGURE 19 shows: (a) the effect of temperature cycling on the CD of GM-CSF in PBS, pH 7.0. GM-CSF at 10°C (―) is compared to GM-CSF which was heated to 90°C and then cooled to 10°C (---); (b) the effect of temperature cycling on the CD of DPPG:PEG-G-CSF (17:1 molar). DPPG:GM-CSF at 10°C (―) is compared to DPPG: GM-CSF which was heated to 90°C and then cooled to 10°C (---).

### DETAILED DESCRIPTION

The compositions prepared in accordance with the present invention are described in more detail in the discussion that follows and are illustrated by the examples provided below. The examples show various aspects of the invention and include results of stability and biological activity testing of various protein:phospholipid compositions. Surprisingly, the interaction of the proteins with the lipid vesicle directly stabilized the protein structure of the protein, thereby exerting a stabilizing effect on the protein even under conditions which lead to denaturation of the protein in the absence of lipid.

Contemplated for use in the practice of the present invention are a variety of proteins capable of transitioning into the molten globular state. Exemplary proteins contemplated are cytokines, including various hematopoietic factors such as the aforementioned G-CSF, GM-CSF, M-CSF, the interferons (alpha, beta, and gamma), the interleukins (1-11), erythropoietin (EPO), fibroblast growth factor, stem cell factor, nerve growth factor, BDNF, NT3, platelet-derived growth factor, and tumor growth factor (alpha, beta). Other proteins may be evaluated for the ability to transition into the MGS. If the protein in question is capable of transitioning into the MGS, the protein in question may then be contacted with a negatively charged liposome vesicle and the stabilizing effects evaluated.

In general, G-CSF useful in the practice of this invention may be a native form isolated pure from mammalian organisms or, alternatively, a product of chemical synthetic procedures or of procaryotic or eucaryotic host expression of exogenous DNA sequences obtained by genomic or cDNA cloning or by gene synthesis. Suitable procaryotic hosts include various bacterial (e.g., *E. coli*) cells. Suitable eucaryotic hosts include yeast (e.g., *S. cerevisiae*) and mammalian (e.g., Chinese hamster ovary, monkey) cells. Depending upon the host employed, the G-CSF expression product may be glycosylated with mammalian or other eucaryotic carbohydrates, or it may be non-glycosylated. The present invention contemplates the use of any and all such forms of G-CSF, although recombinant G-CSF, especially *E. coli* derived, is preferred for reasons of greatest commercial practicality.

The G-CSF to be chemically modified for use in the present invention may also be either natural human G-CSF (nhG-CSF) or the product of a recombinant nucleic acid process, such as prokaryotic or eukaryotic host cell expression. In general, chemical modification contemplated is the attachment of a chemical moiety to the G-CSF molecule itself. A review article describing protein modification and fusion proteins is Francis, *Focus on Growth Factors* 3: 4-10 (May 1992) (published by Mediscript, Mountview Court, Friern Barnet Lane, London N20 OLD, UK). For example, see EP 0 401 384, entitled, *"Chemically Modified Granulocyte Colony Stimulating Factor*," which describes materials and methods for preparing G-CSF to which polyethylene glycol molecules are attached. The attachment may be by bonding, directly to the protein or to a moiety which acts as a bridge to the active agent. Covalent bonding is preferred as the most stable for attachment. The chemical modification may contribute to the controlled, sustained or extended effect of the G-CSF. This may have the effect, for example, of controlling the amount of time the chemically modified G-CSF takes to reach the circulation. An example of a chemical modifier is polyethylene glycol compositions, including derivatives thereof.

Contemplated for use in the practice of this invention are any chemically modified G-CSF preparations which permit efficacy upon administration. Efficacy may be determined by known methods, as a practitioner in the art will recognize. Pegylated G-CSF, especially pegylated *E. coli* derived G-CSF, and more particularly, tri-tetra pegylated *E. coli* derived G-CSF is preferred.

G-CSF has been reported to be most stable under acidic conditions, despite the fact that in the pH range of 2.5 - 5.0, a conformational change occurs which involves a loosening of the tertiary structure and an increase in alpha helical content. Narhi et al., *J. Protein Chem*. 10, 359-367, (1991). This conformational change is characteristic of the molten globular state (MGS). Thus, as is the case for a formulator working with other proteins capable of transitioning into the MGS, a formulator working with G-CSF must protect against thermally-induced unfolding of secondary and tertiary structure in order to prevent aggregation and denaturation.

The GM-CSF useful in the present invention may be a native form isolated pure from mammalian organisms or a product of procaryotic or eucaryotic host expression of exogenous DNA sequences obtained by genomic or cDNA cloning or by gene synthesis. Suitable procaryotic hosts include various bacterial (e.g., *E. coli*) cells. Suitable eucaryotic hosts include yeast (e.g., *S. cerevisiae*) and mammalian (e.g., Chinese hamster ovary, monkey) cells. Depending upon the host employed, the GM-CSF expression product may be glycosylated with mammalian or other eucaryotic carbohydrates, or it may be non-glycosylated. The present invention contemplates the use of any and all such forms of GM-CSF, although recombinant GM-CSF, especially *E. coli* derived, is preferred for reasons of greatest commercial practicality.

The lipid vesicles useful in the compositions of the present invention are those negatively charged liposomes capable of interacting with the protein in question. Particular liposomes contemplated for use include dioleoylphosphatidylglycerol (DOPG), dimyristoylphosphatidylglycerol (DMPG), dipalmitoylphosphatidylglycerol (DPPG), eggphosphatidylglycerol, dioleoylphosphatidylethanolamine (DOPE), eggphosphatidylethanolamine, dioleoylphosphatidic acid (DOPA), dimyristoylphosphatidic acid (DMPA), dipalmitoylphosphatidic acid (DPPA), dioleoylphosphatidylserine (DOPS), dimyristoylphosphatidylserine (DMPS), dipalmitoylphosphatidylserine (DPPS), eggphosphatidylserine, lysophosphatidylglycerol, lysophosphatidylethanolamine, lysophosphatidylserine. Depending on the particular liposome utilized, the amount of liposome could vary.

The protein:phospholipid compositions preferably include a buffering agent to maintain the pH of the solution within a desired range. Preferred agents include sodium acetate, sodium phosphate, and sodium citrate. Mixtures of these buffering agents may also be used. The amount of buffering agent useful in the composition depends largely on the particular buffer used and the pH of the solution. For example, acetate is a more efficient buffer at pH 5 than pH 6 so less acetate may be used in a solution at pH 5 than at pH 6. The preferred pH range for the compositions of the present invention is pH 3.0 - 7.5.

The compositions prepared in accordance with the present invention may further include an isotonicity adjusting agent to render the solution isotonic and more compatible for injection. The most preferred agent is sodium chloride within a concentration range of 0 - 150 mM.

Also comprehended by the invention are pharmaceutical compositions comprising effective amounts of polypeptide products prepared in accordance with the invention together with pharmaceutically acceptable diluents, preservatives, solubilizers, emulsifiers, adjuvants and/or carriers. Such compositions will influence the physical state, stability, and bioavailability of the protein. *See, e.g., Remingtons Pharmaceutical Sciences,* 18th Edition, 1435-1712 (Mack publishing Co., Easton, PA., 1990) which is herein incorporated by reference. What constitutes an effective amount of the protein in a particular case will depend on a variety of factors which the knowledgeable practitioner will take into account, including the desired therapeutic result, the severity of the condition or illness being treated, the physical condition of the subject, and so forth.

In a preferred embodiment involving *E. coli* derived rhG-CSF, the liposome vesicle used is DOPG with a 50:1 ratio of DOPG:G-CSF, at pH 4.5, containing 10 mM sodium acetate.

In a preferred embodiment involving *E. coli* derived rhGM-CSF, the liposome vesicle used is DMPG with a 17:1 ratio of DMPG:GM-CSF, at pH 7.0, in phosphate buffered saline (PBS).

In a preferred embodiment involving *E. coli* derived rhG-CSF which has been chemically modified (pegylated), the rhG-CSF is tri-tetra pegylated, the liposome vesicle used is DMPG with a 17:1 ratio of DMPG:PEG-G-CSF, at pH 4.5.

Although the invention has been described and illustrated with respect to specific protein:lipid compositions and treatment methods, it will be apparent to one of ordinary skill that a variety of related compositions, and treatment methods may exist without departing from the scope of the invention.

The following examples will illustrate in more detail the various aspects of the present invention.

### EXAMPLE 1

Initial experiments were performed to examine the possibility of incorporating recombinant human G-CSF (rhG-CSF) into a lipid vesicle. The rhG-CSF was produced using recombinant DNA technology in which *E. coli* cells were transfected with a DNA sequence encoding human G-CSF as described in U.S. Patent No. 4,810,643 to Souza. The rhG-CSF was prepared as a 4 mg/ml solution in dilute HCl, pH 4.0. All lipids were obtained from Avanti Polar Lipids (Alabaster, Ala) and were stored at -20°C under nitrogen at a final concentration of 100 mg/ml in chloroform.

### Preparation of G-CSF:Phospholipid Complexes

To prepare lipid vesicles for combination with G-CSF, 30 µmole of the appropriate lipid was dispensed into a glass tube and dried to a thin film using a stream of nitrogen gas. The lipid films were desiccated for at least two hours under vacuum to remove any traces of chloroform. The lipid films were hydrated in 1 ml of either distilled de-ionized water (ddH₂O), phosphate buffered saline, pH 7.2 (Gibco/BRL "D-PBS") or 150mM NaCl. The samples were then sonicated in a bath-type sonicator (laboratory Supplies, Hicksville, N.Y.). Sonication was continued until the samples were optically clear (usually between 10-15 minutes). The samples were stored at 4°C under nitrogen until use. The final lipid concentration was 30 mM. Alternatively, the lipid vesicles could be prepared by taking 300 µmole of lipid and drying under nitrogen and dessicating as described above. The dry lipid films were hydrated in 10 ml of appropriate aqueous solution as described above. The samples were then microfluidized in a bench scale emulsifier (Microfluidics Model 110S, Microfluidics, Inc. Cambridge, MA) operating at 7,026,938 kg/m² (10,000 psi). The samples were recycled through the instrument for 10 cycles. The microfluidized samples were then stored at 4°C as described above.

The G-CSF:phospholipid complexes were prepared by mixing G-CSF (as described above) with a particular lipid (as described above). Mixing was accomplished by vortexing, stirring, or gentle shaking. Various mole ratios of lipid:G-CSF were prepared to evaluate membrane insertion and stabilization of protein. For example, to prepare a 3 ml sample (in water) which is 0.2 mg/ml G-CSF at a 40:1 mole ratio of lipid:G-CSF, 150 µl of G-CSF stock is combined with 44 µl of lipid (30 mM stock, prepared in water by sonication) and water is added to achieve a final sample volume of 3 ml. A five minute incubation is recommended (but not necessary), and was used here before using or assaying the sample.

G-CSF can also be combined with the hydrated lipid prior to microfluidization. Subsequent microfluidization of the mixtures as described above leads to incorporation of G-CSF into the lipid membrane.

### Analysis of the G-CSF: Phospholipid Complexes

### 1. Tryptophan emission spectra.

There are two tryptophan residues in rhG-CSF that are quite sensitive to local environmental conditions. Therefore, analysis was performed to determine the rhG-CSF tryptophan fluorescence when the rhG-CSF is contacted with a liposome. A blue shift in fluorescence emission maximum would suggest chat the tryptophans are in a more hydrophobic environment and therefore the rhG-CSF was embedded in the lipid membranes. An excellent review of tryptophan fluorescence analysis is *Principles of Fluorescence Microscopy,* by J. Lakowicz, Chap 11 (Plenum Press, New York, 1983).

Tryptophan fluorescence of the G-CSF:lipid complexes (as described above) was assayed by exciting the samples at 280 nm and while scanning the emission from 285 nm to 420 nm in 1nm increments at a rate of 1 nm/sec. The sample volume was 3 ml and the final concentration of G-CSF was 0.2 mg/ml for all samples. The lipid:G-CSF ratios varied. All fluorescence measurements were carried out using a PTI Alphascan fluorometer (South Brunswick, NJ). All measurements were performed at 25°C and this temperature was maintained through the use of a water-jacketed cuvette holder connected to a circulating water bath. Emission spectra were collected and analyzed using the data software provided by PTI.

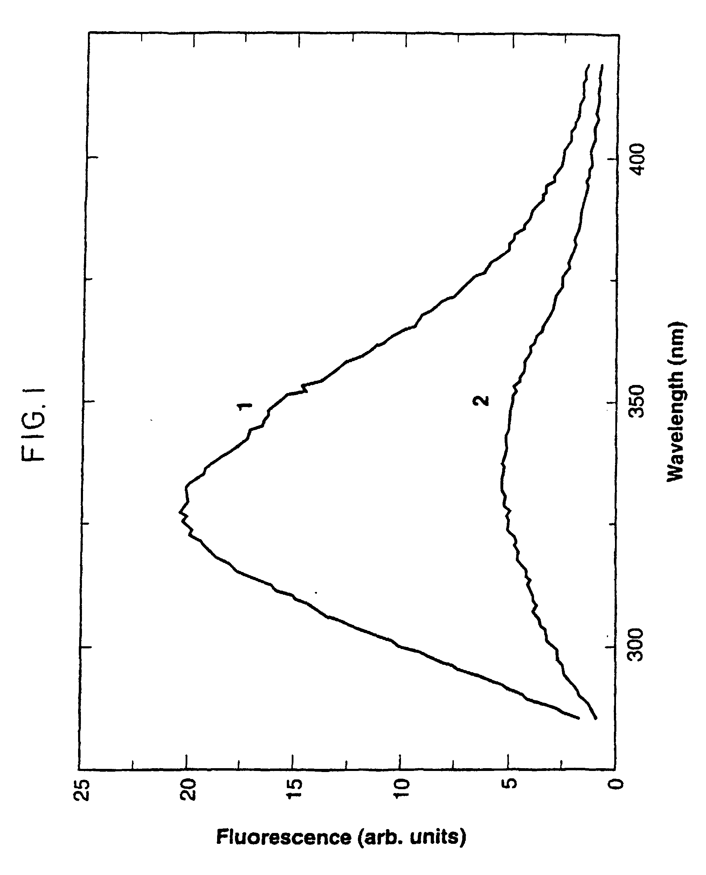

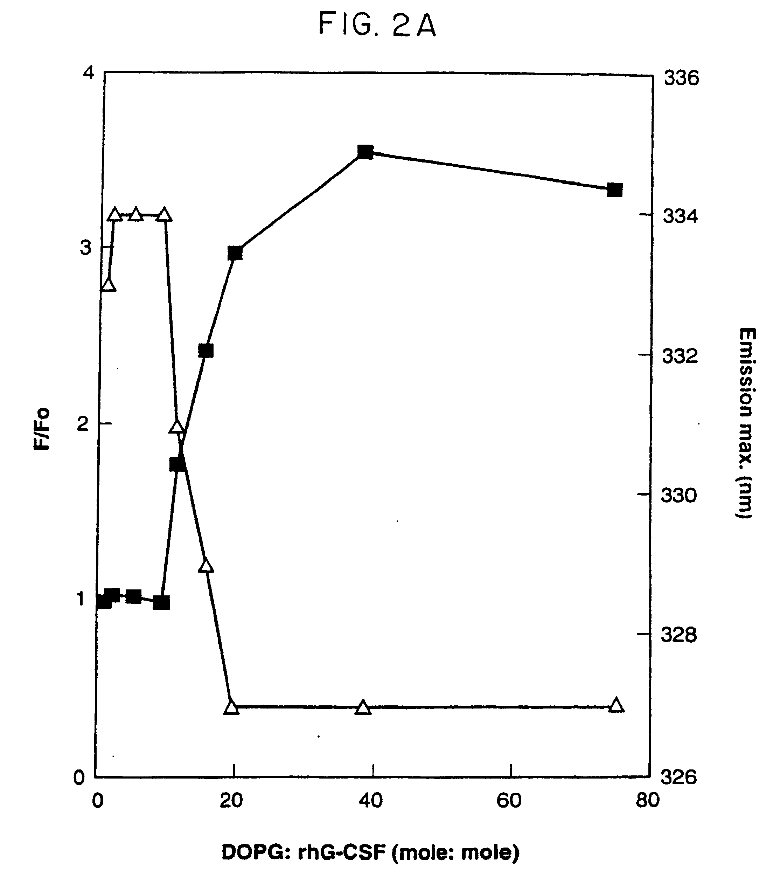

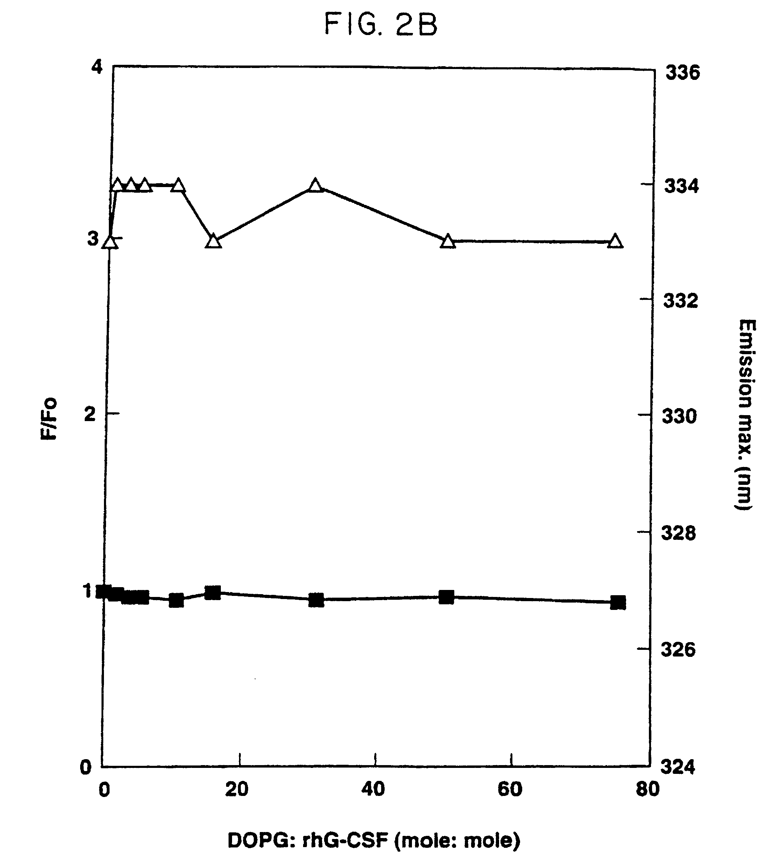

The fluorescence spectra of rhG-CSF in the presence and absence of small unilamellar vesicles composed of DOPG is shown in Figure 1. rhG-CSF has an emission maximum at 334 nm in the absence of DOPG vesicles. In the presence of DOPG at a 100:1 lipid:protein ratio, rhG-CSF tryptophan fluorescence exhibits a blue shift in fluorescence emission maximum to 327 nm and a dramatic increase in fluorescence intensity. The low wavelength of the fluorescence emission in the presence of DOPG suggests that the tryptophans are in an environment more hydrophobic than the native protein. As demonstrated in Figure 2, the fluorescence shifts depend on the mole ratio of DOPG:G-CSF and membrane insertion is detectable once a 10:1 ratio of DOPG:G-CSF is reached.

### 2. Iodide quenching experiments.

Iodide is an efficient collisional quencher of tryptophan fluorescence, but cannot penetrate lipid membranes. Therefore, efficient quenching of tryptophan fluorescence by iodide indicates exposure of the residues to the bulk aqueous solvent while protection from iodide quenching occurs when protein tryptophans are sequestered away from the aqueous solvent. In these experiments, G-CSF and a DOPG:G-CSF composition (100:1 lipid:protein ratio) was used. After initial readings (F_{O}) on the samples were taken and recorded, fluorescence intensity was measured after addition of increasing amounts of potassium iodide (KI) (5M stock). Both the sample and KI solutions were prepared to contain 1 mM Na₂SO₃ (final concentration) as described by Lee et al, *Biochem. Biophys. Acta*, 984: 174-182 (1989) and Le Doan et al., *Biochem. Biophys. Acta*, 858: 1-5 (1986). The addition of Na₂SO₃ prevents the formation of I₂ which can partition into nonpolar regions of proteins and membranes. The data were analyzed by the Stern-Volmer equation (F_{O}/F = 1 + K_{KI}[KI]), where F_{O} and F are the fluorescence intensities of samples in the absence and presence, respectively, of KI at concentration [KI]. K_{KI} is the Stern-Volmer quenching constant for KI quenching of G-CSF tryptophan residues; Lehrer, S., *Biochemistry* 10: 3254-3263 (1979).

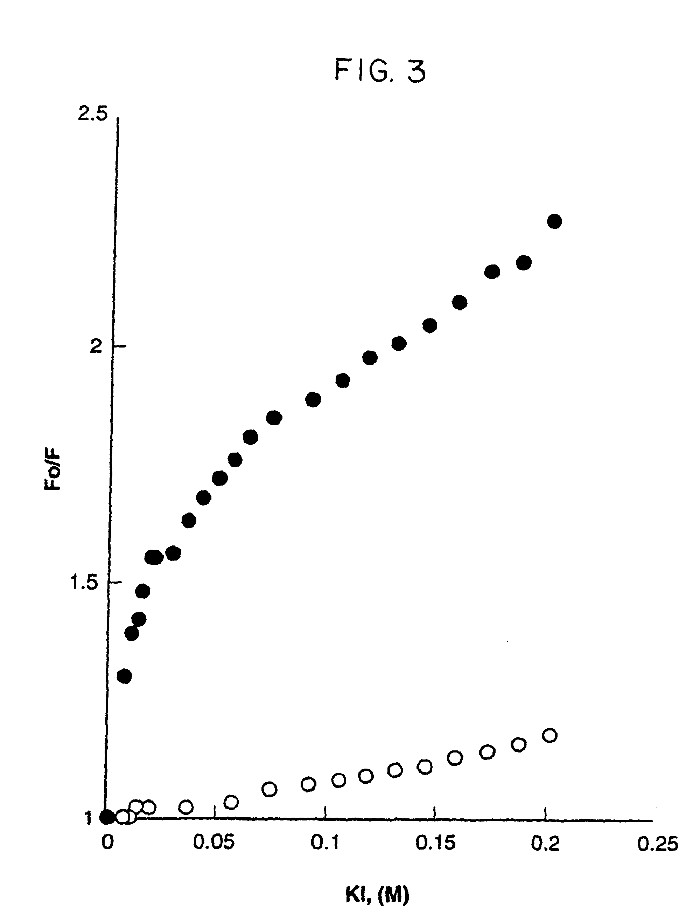

The Stern-Volmer plots of the data are shown in Figure 3. In the absence of DOPG vesicles, rhG-CSF fluorescence is efficiently quenched by KI. In the presence of DOPG, the Stern-Volmer plot of the data is linear, indicating that iodide has poor access to both tryptophans. The data shows that the tryptophan residue which is iodide-accessible in the absence of DOPG becomes iodide-inaccessible in the presence of DOPG. Therefore, the portion of rhG-CSF containing this tryptophan must be embedded in the DOPG bilayer.

### 3. Energy transfer measurements.

As previously shown, energy transfer can occur between tryptophan donors and lipid soluble fluorescent acceptors such as pyrene decanoic acid, since the excitation spectrum of this probe significantly overlaps the emission spectra of tryptophan. Friere et al., *Biochemistry* 22: 1675-1680 (1983). If the protein inserts into lipid membranes, energy transfer from tryptophan to pyrene will lead to quenching of the tryptophan fluorescence. In this experiment, the tryptophan emission intensity of various G-CSF:lipid complexes was recorded before (F_{O}) and after (F) addition of various amounts of pyrene decanoic acid (30 µg/ml stock in tetrahydrofuran). The samples were stirred continuously during pyrene decanoic acid addition to promote mixing between pyrene decanoic acid and the sample. The ratio of F/F_{O} is proportional to the amount of energy transfer occurring between G-CSF tryptophans and the hydrophobic energy acceptor pyrene decanoic acid.

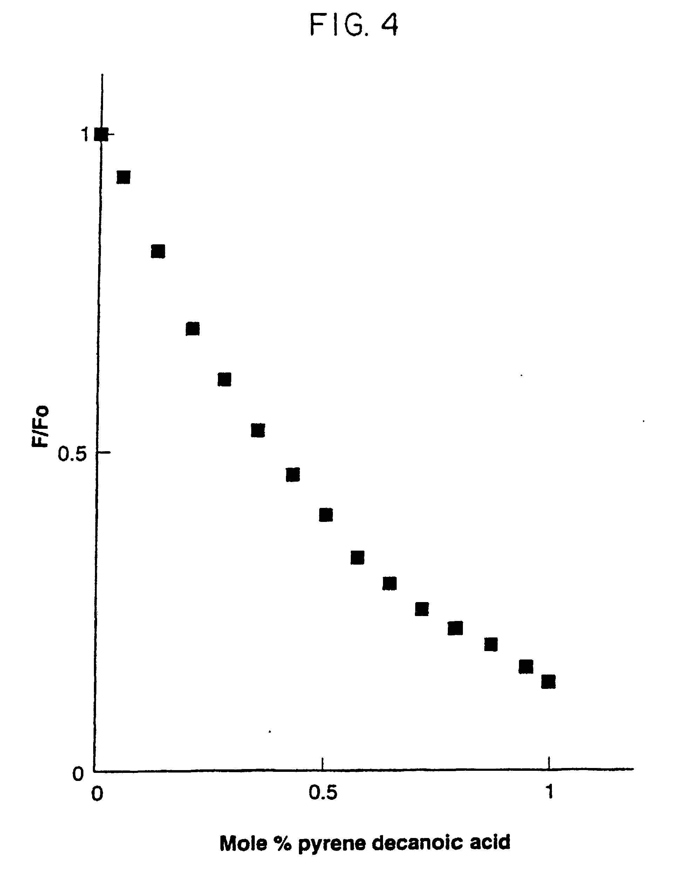

Figure 4 shows the quenching profile for rhG-CSF in the presence of DOPG (100:1 lipid:protein ratio) as a function of added pyrene decanoic acid. The quenching occurs at very low pyrene decanoic acid concentrations (<1 mole %), so the effect of the fluorescent probe on the membrane structure and behavior is minimal. Since pyrene decanoic acid can be expected to rapidly partition into lipid bilayers, the present data indicate that rhG-CSF is embedded in DOPG membranes deep enough to allow efficient energy transfer from tryptophan to the pyrene acceptor. Energy transfer was confirmed by examining the excitation spectra of pyrene decanoic acid-labeled DOPG vesicles in the presence and absence of rhG-CSF.

The analysis above shows that rhG-CSF can closely interact with an unsaturated phospholipid like DOPG. In the presence of DOPG vesicles, a rhG-CSF tryptophan is protected from a water soluble fluorescence quencher but is susceptible to quenching via energy transfer to a hydrophobic fluorescent probe. Taken together, the data show that rhG-CSF can insert into membranes composed of DOPG. Membrane insertion is detectable once a 10:1 ratio (lipid:G-CSF) is reached, and this number may represent the number of lipids which surround the inserted portion of the protein.

### EXAMPLE 2

In this example, the ability of rhG-CSF to interact with other phospholipids was determined using comparisons of F/F_{O} intensity and emission maximums as described above. In each instance, the mole ratio of lipid:rhG-CSF was 100:1.

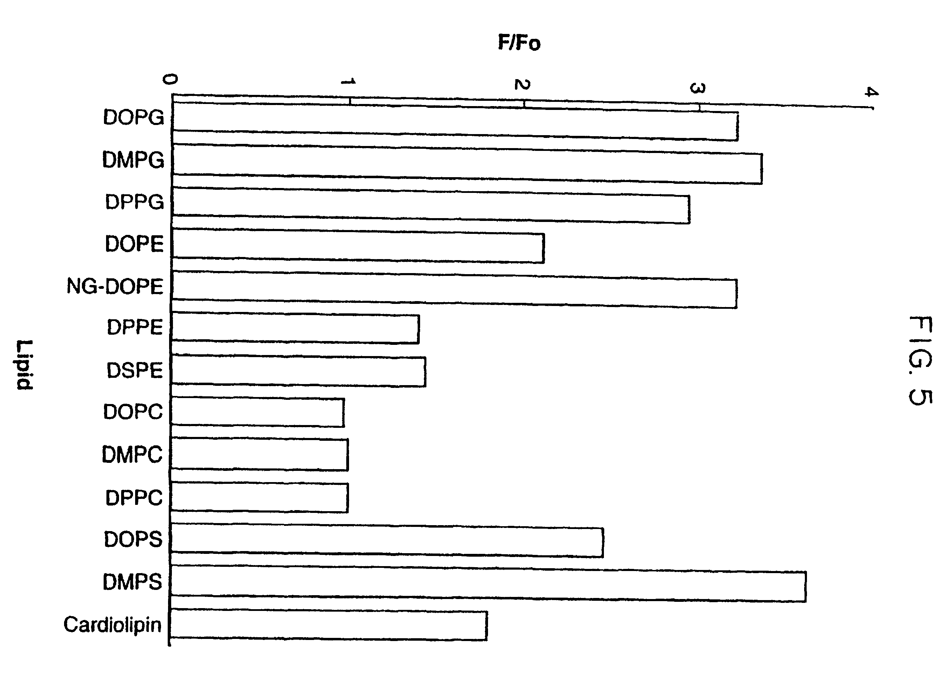

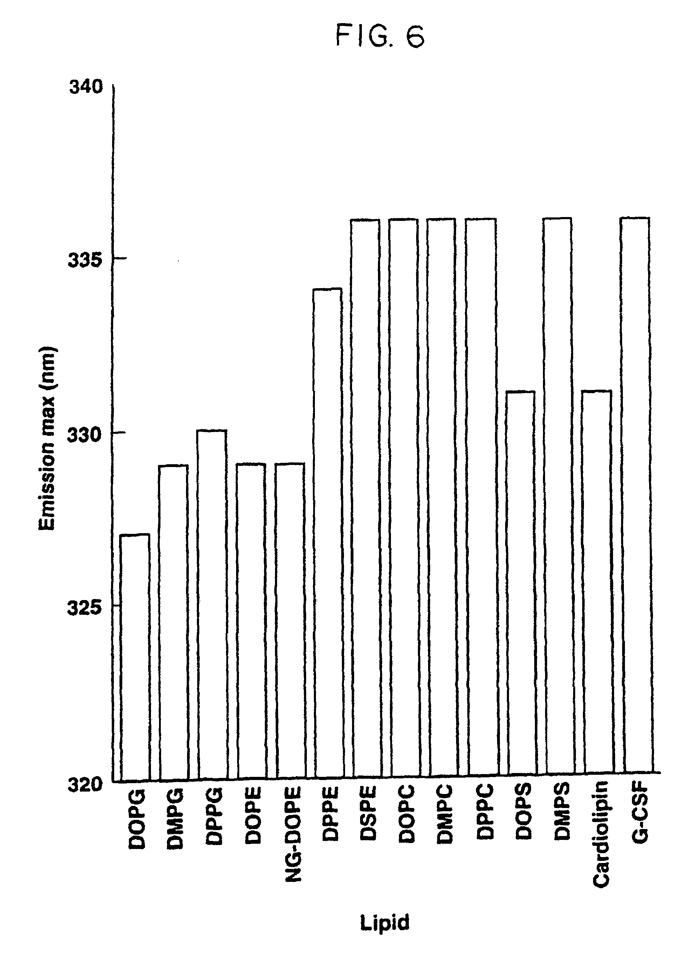

Figure 5 shows the F/F_{O} data for rhG-CSF in the absence and presence of various lipids. Figure 6 shows the emission maximum data for the same compositions. The data in Figure 5 and Figure 6 demonstrates that, in addition to DOPG, rhG-CSF can insert into DMPG, DPPG, and less efficiently, into the phosphatidylethanolamines (PE's) and the phosphatidylserines (PS's). In addition, NG-DOPE (DOPE sample where the PE headgroup was made more negative) was found to provide for improved insertion of the rhG-CSF than DOPE.

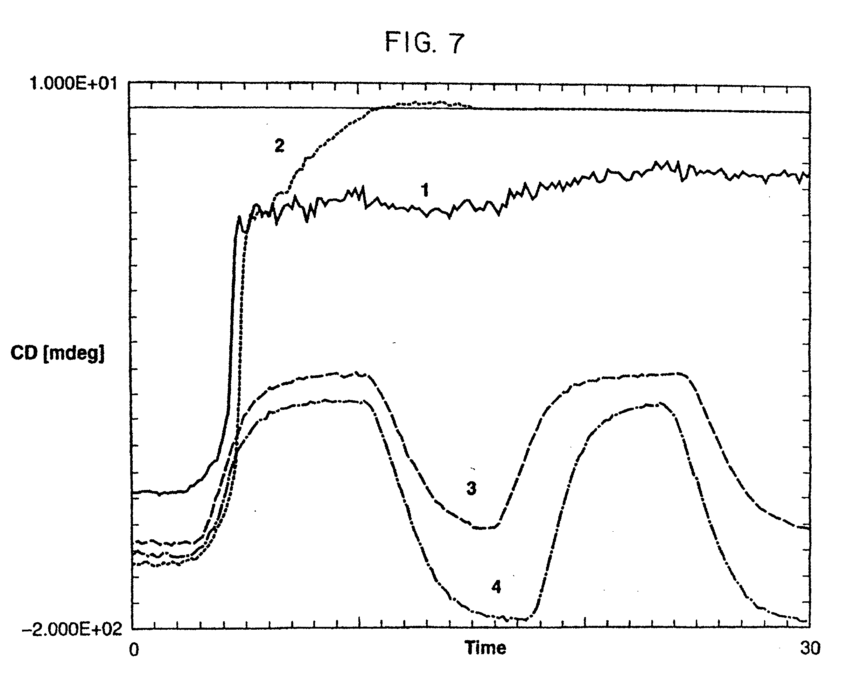

DOPC, DMPC and DPPC are neutral lipids and these vesicles had little if no effect on either the emission maximum or the fluorescence intensity of rhG-CSF, indicating that no interaction took place with these phospholipids (see Figure 5 and 6, and Figure 7, curve 2).

The data above demonstrate that a protein capable of transitioning into the molten globular state can insert in various lipid vesicles. However, this rhG-CSF:lipid interaction only occurs when a negatively charged lipid vesicle is used. Among the negatively charged lipid vesicles, those vesicles with the greater negative charge seem to provide for a stronger rhG-CSF:lipid interactions.

### EXAMPLE 3

In this example, the effect of the rhG-CSF:DOPG interaction as it relates to protein stability was determined. Circular dichroism measurements were made on a Jasco J-720 instrument equipped with a Peltier-type thermostatted cell holder and a magnetic stirrer. Circular dichroism at 222 nm was measured using a final rhG-CSF concentration of 80 µg/ml, pH 6.0. Differential scanning calorimetry measurements were made using a Microcal MC-2 calorimeter. Samples of rhG-CSF (1 mg/ml, in water) or DOPG:rhG-CSF (45:1 mole/mole, in water) were scanned at a rate of 90°C/hour. Data were stored and analyzed using the Microcal software.

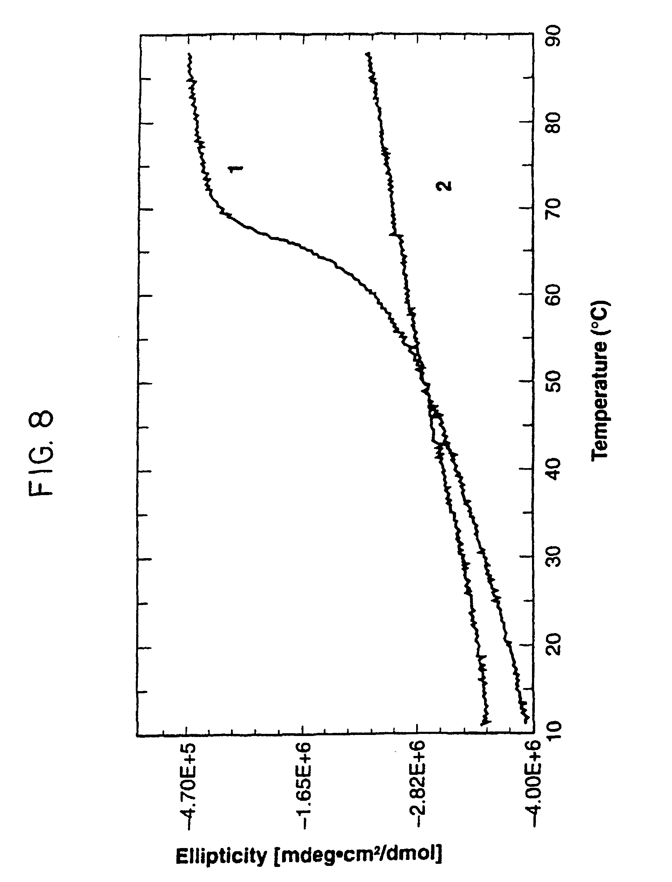

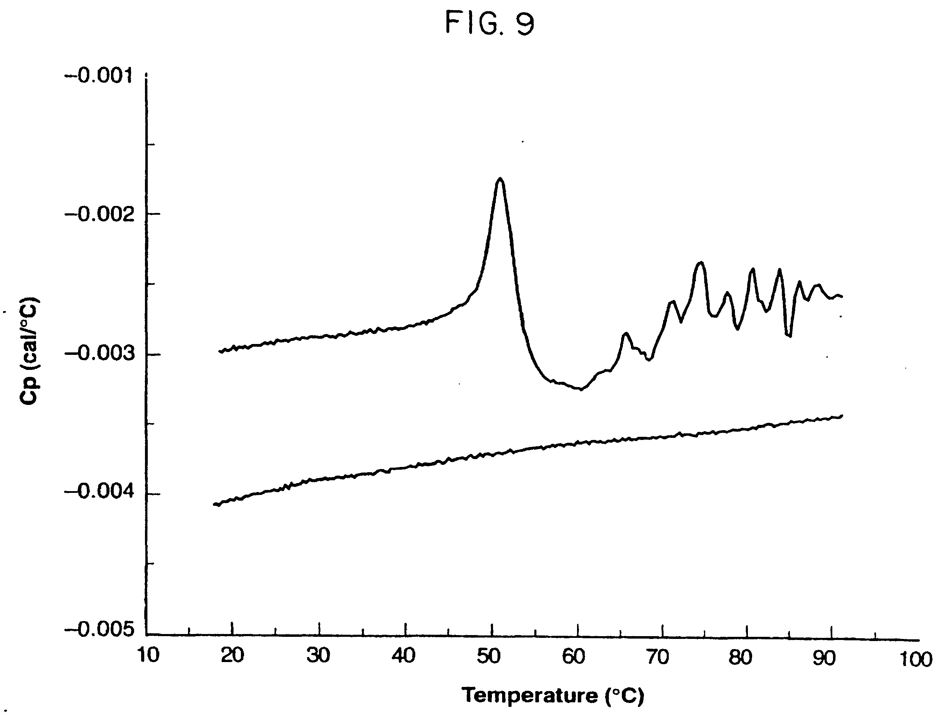

Temperature-induced changes in the alpha helicity of G-CSF can be followed by measuring circular dichroism (222 nm) as a function of increasing temperatures. The thermally-induced unfolding of rhG-CSF at pH 6.0 is shown in Figure 8. The curve indicates that a fairly sharp transition occurs at ∼60-70°C which leads to loss of alpha helicity. After this transition the rhG-CSF irreversibly precipitates from solution. The temperature range of the unfolding is similar to the melting temperature of rhG-CSF at pH 7.0 as determined by differential scanning calorimetry as shown in Figure 9.

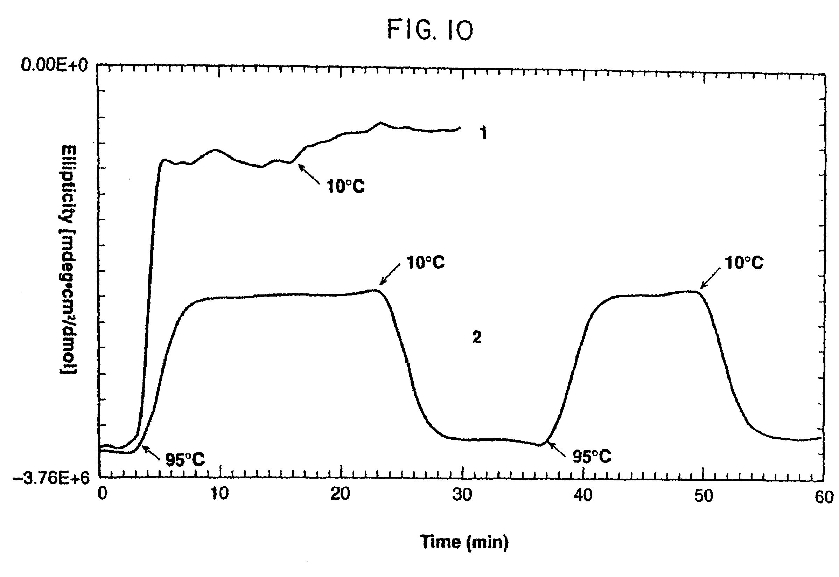

By contrast, DOPG:rhG-CSF samples show a gradual loss of alpha helicity with increasing temperature and, unlike rhG-CSF alone, the temperature-induced unfolding of DOPG:rhG-CSF does not appear to be cooperative (see Figure 8). This conclusion is also demonstrated by the lack of a melting transition as shown by differential scanning calorimetry (Figure 9). Remarkably, DOPG:rhG-CSF samples can recover alpha helicity after heating to 95°C and can be repeatedly cycled between 95°C and 10°C with full recovery of helicity upon cooling (see Figure 10). rhG-CSF alone under these conditions is irreversibly unfolded and precipitates from solution.

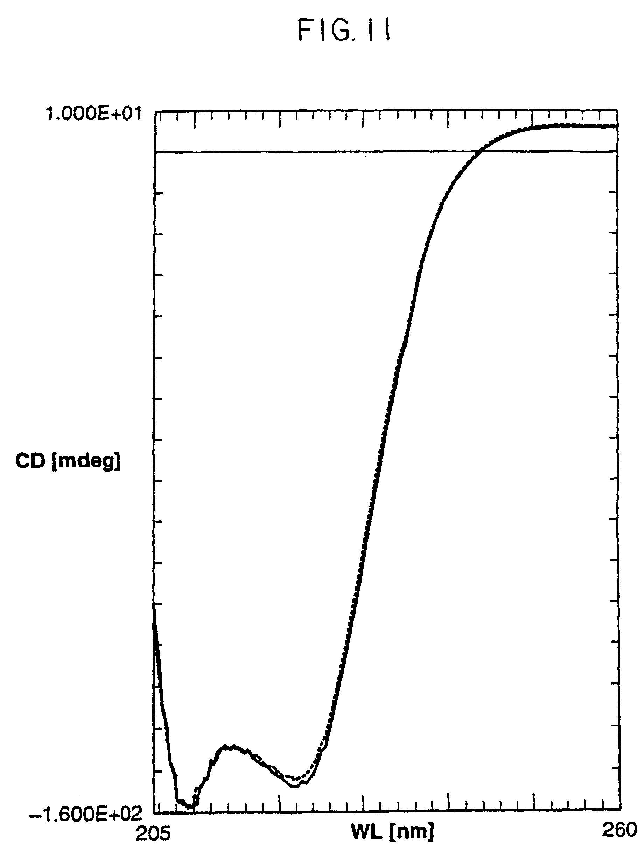

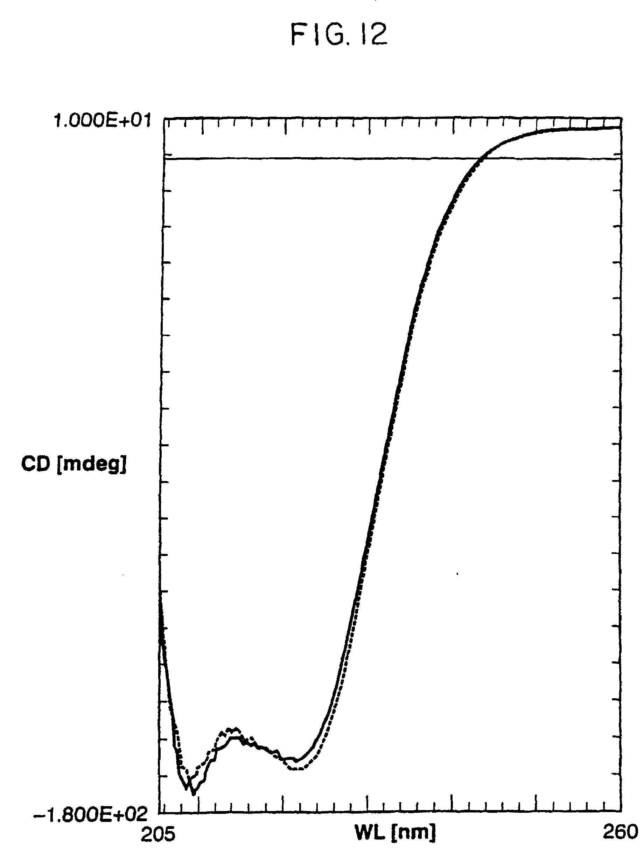

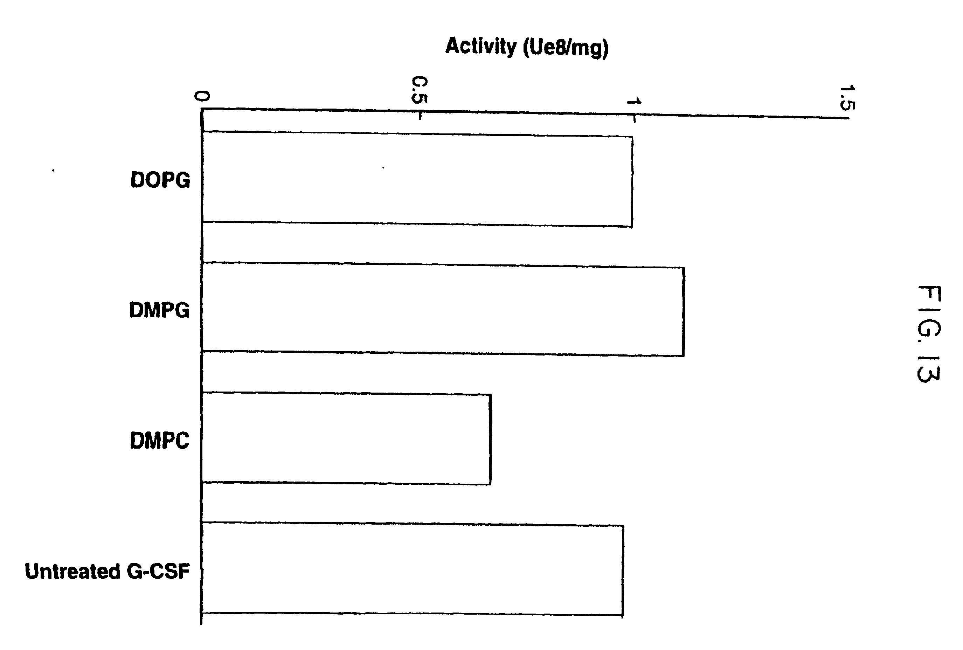

The effects of DMPG and DPPG on G-CSF circular dichroism was also examined. A 150:1 lipid:rhG-CSF ratio was used and, as was the case with DOPG, DMPG and DPPG also stabilize the secondary structure of rhG-CSF (Figures 11-13).

These data demonstrate that the interaction of rhG-CSF with DOPG, DMPG and DPPG enhances the stability of the protein under conditions where rhG-CSF alone is unstable. The interaction directly stabilizes the secondary and tertiary structure of rhG-CSF.

### EXAMPLE 4

In this example, the effect of the rhG-CSF:DOPG interaction as it relates to the biological activity of rhG-CSF was determined. The *in vitro* activity of rhG-CSF was assayed utilizing the G-CSF dependent uptake of [³H]-thymidine by murine bone marrow cells as described in Zsebo et al., *Immunobiology* 172: 175-184 (1986). All activity assays were performed in triplicate. *In vivo* activity was determined by subcutaneous injection of hamsters (rhG-CSF dose of 100 µg/kg) and measurements of white blood cell (WBC) count.

### 1. In vitro activity

A. The specific activity of rhG-CSF in the absence and presence of DOPG was determined. Heat treated rhG-CSF and DOPG:rhG-CSF samples were also tested. The results are summarized in Table 1.

**TABLE 1**

| Sample | Specific activity (U/mg/protein) |
|---|---|
| rhG-CSF | 0.66 ± 0.09 |
| rhG-CSF (heated)^{a} | Not Detectable |
| DOPG:rhG-CSF^{b} | 0.61 ± 0.11 |
| DOPG:rhG-CSF^{b} (heated)^{a} | 0.52 ± 0.08 |

| | |
|---|---|
| ^{a}Sample was incubated for 10 minutes at 85°C in a water bath prior to performing the assay. | |
| ^{b}DOPG:rhG-CSF ratio of 50:1 (mole/mole). | |

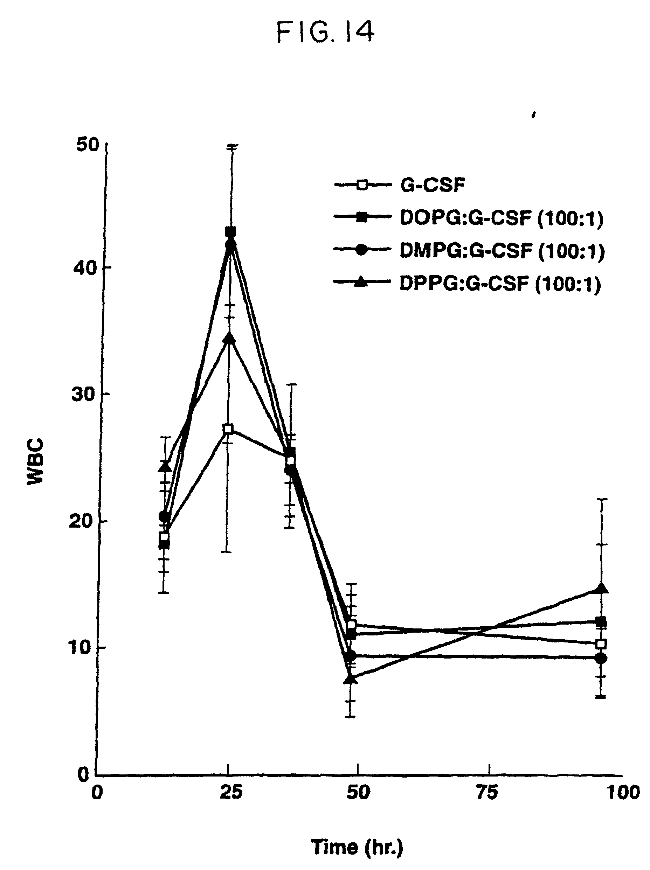

As shown in Table 1, insertion into DOPG bilayers does not adversely affect the biological activity of rhG-CSF. After heating to 85°C for 10 minutes, rhG-CSF has undetectable activity and the protein precipitates. After similar treatment, DOPG:rhG-CSF retains ∼85% of the activity of unheated rhG-CSF and fully recovers secondary structure upon cooling.
B. The ability of various lipids to stabilize rhG-CSF during freeze drying was also studied. Samples of rhG-CSF in combination with various lipids were freeze dried and assayed (as described above) for activity. DOPG, DMPG, and DPPG when mixed with rhG-CSF allow ∼100% retention of rhG-CSF bioactivity after freeze drying (Figure 14). rhG-CSF alone did not survive the freeze-drying process.

### 2. In vivo activity

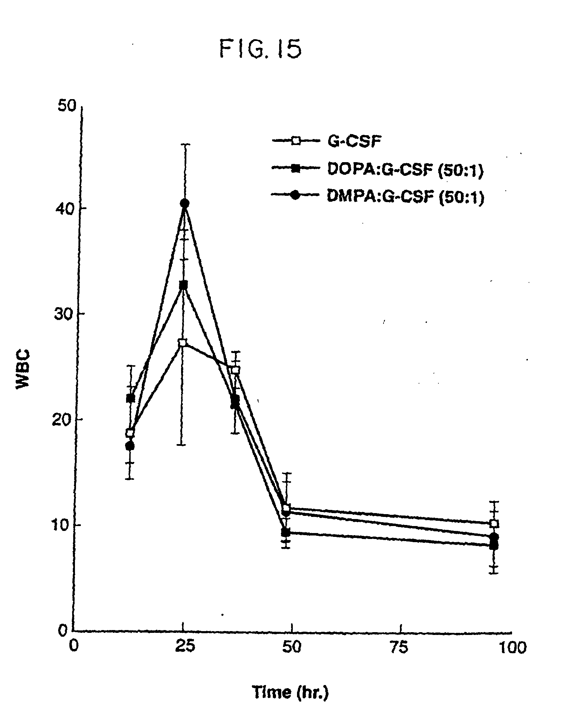

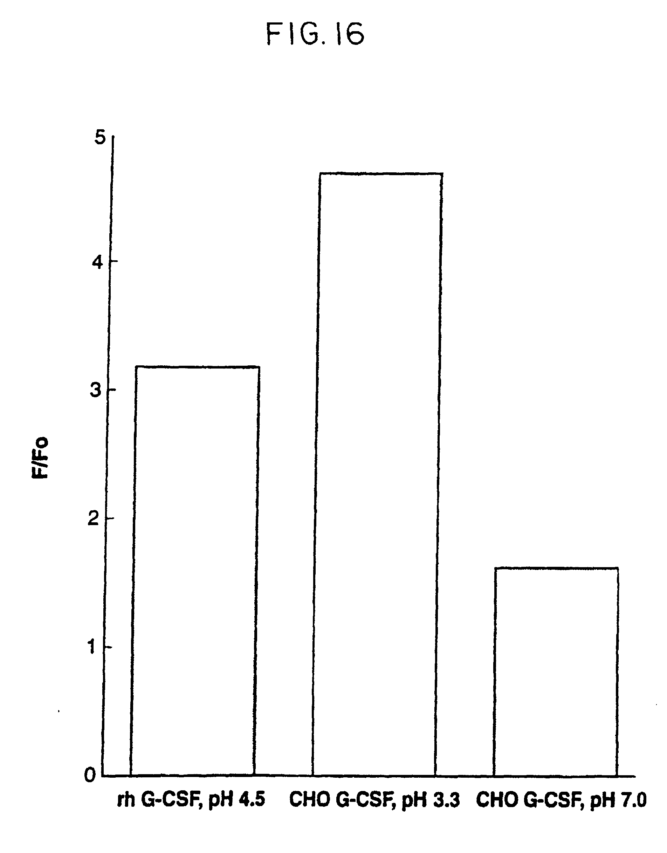

The activity (WBC count) of rhG-CSF in the absence and presence of lipid was determined. Activity was measured after subcutaneous injection (rhG-CSF dose of 100 µg/kg) on day 0. Five different lipid:rhG-CSF complexes were assayed and in each instance the lipid:rhG-CSF complex retained *in vivo* activity (Figures 15 and 16).

The studies above demonstrate that insertion into negatively charged lipid bilayers does not adversely affect the biological activity of rhG-CSF. Additionally, it appears that the protective effect of the lipid protects rhG-CSF during the freeze-drying process.

### EXAMPLE 5

In this example, chemically modified G-CSF (pegylated G-CSF (PEG-G-CSF)) and G-CSF obtained as a product of eucaryotic host cell expression (CHO-G-CSF) were tested for their ability to interact with negatively charged lipid vesicles. For the CHO-G-CSF, the determinations were made using comparisons of F/F_{O} intensity and emission maximums (as described in Example 1 above). In each instance, the mole ratio of lipid:protein was 100:1. For the PEG-G-CSF, the determination was based upon circular dichroism analysis.

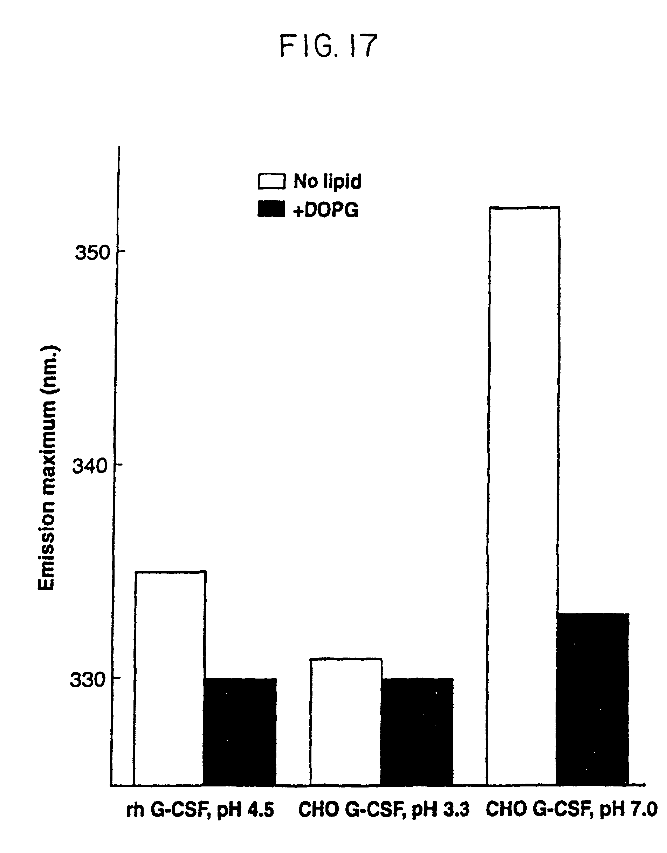

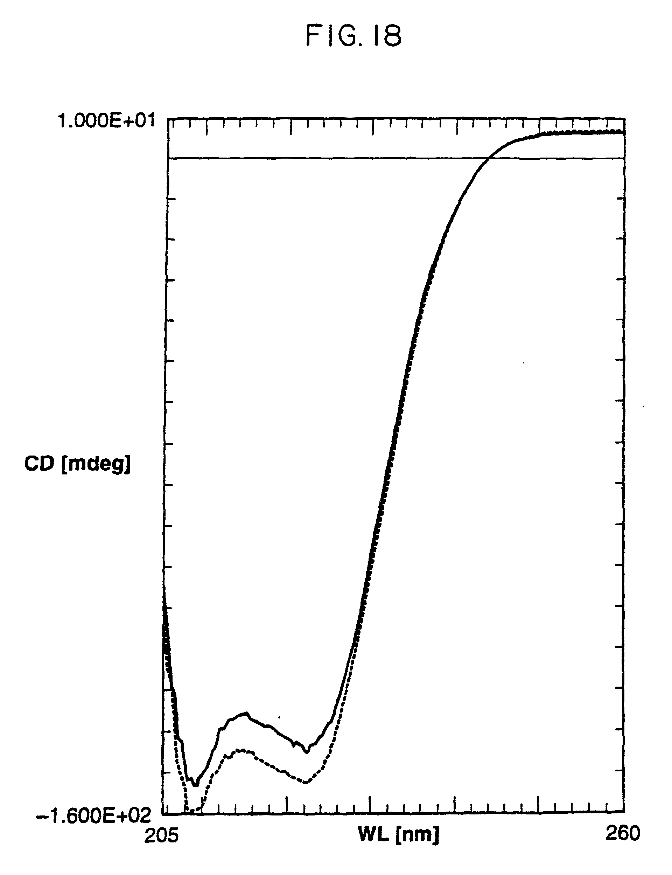

The CHO-G-CSF used was produced using recombinant DNA technology in which Chinese Hamster Ovary (CHO) cells were transfected with a DNA sequence encoding human G-CSF as described in U.S. Patent No. 4,810,643 to Souza. The CHO-G-CSF was prepared as a .6 mg/ml solution in PBS, pH 7.0. CHO-G-CSF was shown to interact with DOPG in a manner similar to rhG-CSF, with each sample showing increased fluorescence intensity in the presence of DOPG, as well as a blue shift in emission maximum in the presence of DOPG (Figures 17 and 18). Therefore, the DOPG interaction is not due to some peculiarity of the recombinant form of G-CSF.

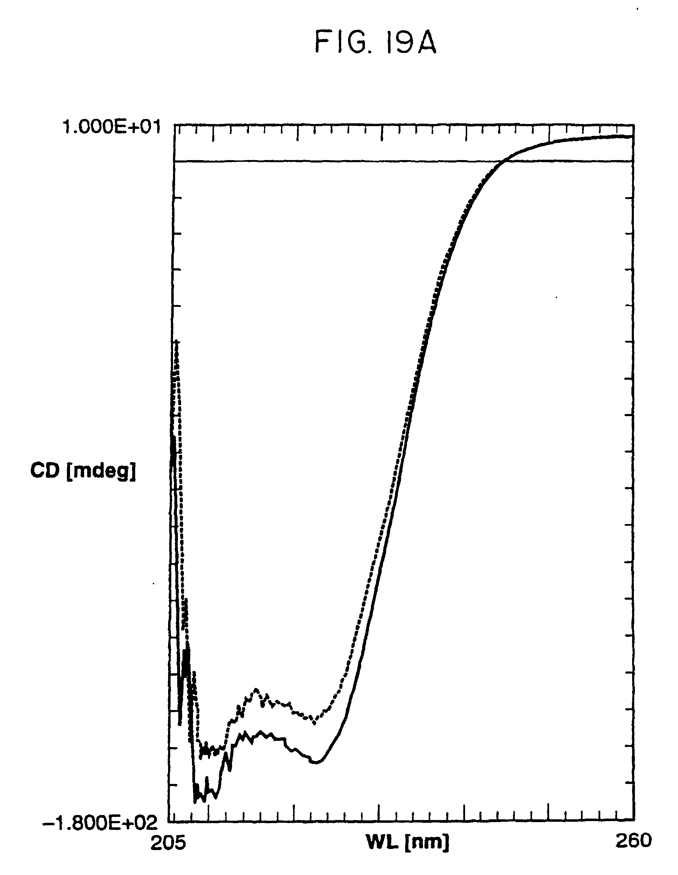

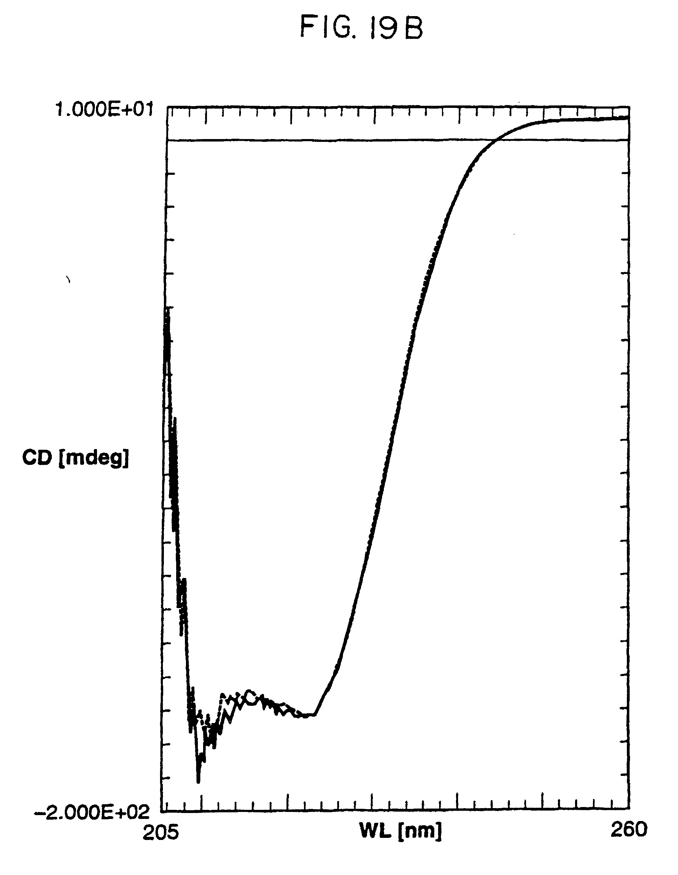

PEG-G-CSF used in these experiments was tri-tetra pegylated *E. coli* derived G-CSF (using PEG 6000). DMPG:PEG-G-CSF (17:1 mole/mole) samples were prepared using procedures described above. The DMPG:PEG-G-CSF samples were found to fully recover secondary structure after heating (Figure 19). Despite the presence of the PEG molecules, the derivatized protein was able interact with the lipid in the same way as the native protein.

The data above show that the stabilizing effects associated with G-CSF interaction with a negatively charged lipid vesicle is not unique only to rhG-CSF obtained as a product of procaryotic host cell expression. A chemically modified protein capable of transitioning to a MGS and contacted with a liposome vesicle, here PEG-G-CSF:DMPG, also exhibited stabilizing effects.

### EXAMPLE 6

In this example, the effects of DMPG and DPPG on GM-CSF was studied. The GM-CSF was recombinant human GM-CSF as described in U.S. Patent No. 5,047,504 to Boone, and prepared as a 1 mg/ml solution in phosphate buffered saline (PBS), pH 7.0. A lipid:GM-CSF ratio of 17:1 was used and thermal stability was measured using circular dichroism analysis as described above. DMPG and DPPG can lead to better thermal stability of GM-CSF, i.e., recovery of secondary structure after heating (Figures 20a & 20b).

These data provide another example of a protein, capable of transitioning into the molten globular state, interacting with a negatively charged lipid vesicle to provide better thermal stability to the protein.

## Claims

1. A process for preparing a pharmaceutical composition in which a protein is stabilised against unfolding of the secondary structure of the protein which process comprises contacting the protein, which protein is capable of transitioning into the molten globular state, with a negatively charged liposome vesicle, wherein the liposome-protein complex directly stabilises against unfolding of the secondary structure of the protein, and obtaining a liposome-protein pharmaceutical composition.

2. A process according to Claim 1, wherein said composition has a pH of from 3.0 to 7.5 and has at least a 10:1 ratio of lipid:protein.

3. A process according to Claim 1, wherein said liposome vesicle is selected from
dioleoylphosphatidylglycerol (DOPG),
dimyristoylphosphatidylglycerol (DMPG),
dipalmitoylphosphatidylglycerol (DPPG),
eggphosphatidylglycerol,
dioleoylphosphatidylethanolamine (DOPE),
eggphosphatidylethanolamine, dioleoylphosphatidic acid (DOPA), dimyristoylphosphatidic acid (DMPA),
dipalmitoylphosphatidic acid (DPPA),
dioleoylphosphatidylserine (DOPS),
dimyristoylphosphatidylserine (DMPS),
dipalmitoylphosphatidylserine (DPPS),
eggphosphatidylserine, lysophosphatidylglycerol,
lysophosphatidylethanolamine, and
lysophosphatidylserine.

4. A process according to Claim 1, 2 or 3, wherein said protein is a cytokine.

5. A process according to Claim 4, wherein said cytokine is a hematopoietic factor.

6. A process according to Claim 5, wherein said hematopoietic factor is selected from G-CSF and GM-CSF.

7. A process according to Claim 5, wherein said hematopoietic factor is G-CSF.

8. A process according to Claim 7, wherein the G-CSF is natural human G-CSF or is obtained as a product of procaryotic or eukaryotic host cell expression.

9. A process according to Claim 7, wherein the G-CSF is chemically modified G-CSF.

10. A process according to Claim 9, wherein the chemically modified G-CSF is pegylated G-CSF.

11. A process according to Claim 1, wherein said protein is *E. coli* derived rhG-CSF, wherein said liposome vesicle is DOPG, and wherein said composition has a 50:1 ratio of DOPG:rhG-CSF, has a pH of 4.5 and contains 10 mM sodium acetate.

## Patentansprüche

1. Verfahren zur Herstellung einer pharmazeutischen Zusammensetzung, in der ein Protein gegen Auseinanderfalten der Sekundärstruktur des Proteins stabilisiert ist, wobei das Verfahren das in Kontakt bringen des Proteins, wobei das Protein in der Lage ist, in den geschmolzenen globulären Zustand überzugehen, mit einem negativ geladenen Lioposomvesikel umfaßt, wobei der Liposom-Protein-Komplex direkt gegen ein Auseinanderfalten der Sekundärstruktur des Proteins stabilisiert, und Erhalten einer Liposom-Protein-pharmazeutischen Zusammensetzung.

2. Verfahren nach Anspruch 1, wobei die Zusammensetzung einen pH von 3,0 bis 7,5 aufweist und mindestens ein 10:1-Verhältnis von Lipid : Protein aufweist.

3. Verfahren nach Anspruch 1, wobei das Liposomvesikel ausgewählt ist aus
Dioleoylphosphatidylglycerin (DOPG),
Dimyristoylphosphatidylglycerin (DMPG),
Dipalmitoylphosphatidylglycerin (DPPG),
Eiphosphatidylglycerin,
Dioleoylphophatidylethanolamin (DOPE),
Eiphosphatidylethanolamin, Dioleoylphosphatidsäure (DOPS), Dimyristoylphosphatidsäure (DMPS),
Dipalmitoylphosphatidsäure (DPPS),
Dioleoylphosphatidylserin (DOPS),
Dimyristoylphosphatidylserin (DMPS),
Dipalmitoylphosphatidylserin (DPPS),
Eiphosphatidylserin, Lysophosphatidylglycerin,
Lysophosphatidylethanolamin und
Lysophosphatidylserin.

4. Verfahren nach Anspruch 1, 2 oder 3, wobei das Protein ein Cytokin ist.

5. Verfahren nach Anspruch 4, wobei das Cytokin ein hämatopoetischer Faktor ist.

6. Verfahren nach Anspruch 5, wobei der hämatopoetische Faktor ausgewählt ist aus G-CSF und GM-CSF.

7. Verfahren nach Anspruch 5, wobei der hämatopoetische Faktor G-CSF ist.

8. Verfahren nach Anspruch 7, wobei der G-CSF ein natürlicher menschlicher G-CSF ist oder als ein Produkt von prokaryontischer oder eukaryontischer Wirtszellexpression erhalten ist.

9. Verfahren nach Anspruch 7, wobei der G-CSF chemisch modifizierter G-CSF ist.

10. Verfahren nach Anspruch 9, wobei der chemisch modifizierte G-CSF pegylierter G-CSF ist.

11. Verfahren nach Anspruch 1, wobei das Protein *E. coli*-abstammender rhG-CSF ist, wobei das Liposomenvesikel DOPG ist und wobei die Zusammensetzung ein 50:1 Verhältnis von DOPG : rhG-CSF aufweist, einen pH von 4,5 aufweist und 10 µM Natriumacetat enthält.

## Revendications

1. Procédé pour préparer une composition pharmaceutique dans laquelle une protéine est stabilisée contre le déplissement de la structure secondaire de la protéine, lequel procédé consiste à mettre en contact la protéine, laquelle protéine est capable de transition dans l'état globulaire fondu, avec une vésicule liposome chargée négativement, où le complexe liposome-protéine stabilise directement contre le déplissement de la structure secondaire de la protéine, et à obtenir une composition pharmaceutique liposome-protéine.

2. Procédé selon la revendication 1, dans lequel ladite composition à un pH de 3,0 à 7,5 et présente au moins un rapport 10 : 1 de lipide : protéine

3. Procédé selon la revendication 1, dans lequel ladite vésicule liposome est choisie à partir de :
dioléoylphosphatidylglycérol (DOPG),
dimyristoylphosphatidylglycérol (DMPG),
dipalmitoylphosphatidylglycérol (DPPG),
eggphosphatidylglycérol,
dioléoylphosphatidyléthanolamine (DOPE),
eggphosphatidyléthanolamine, acide dioléoylphosphatidique (DOPA),
acide dimyristoylphosphatidique (DMPA),
acide dipalmitoylphosphatidique (DPPA),
dioléoylphosphatidylsérine (DOPS),
dimyristoylphosphatidylsérine (DMPS),
dipalmitoylphosphatidylsérine (DPPS),
eggphosphatidylsérine, lysophosphatidylglycérol,
lysophosphatidyléthanolamine, et
lysophosphatidylsérine.

4. Procédé selon la revendication 1, 2 ou 3, dans lequel ladite protéine est une cytokine.

5. Procédé selon la revendication 4, dans lequel ladite cytokine est un facteur hématopoïétique.

6. Procédé selon la revendication 5, dans lequel ledit facteur hématopoïétique est choisi à partir de G-CSF et GM-CSF.

7. Procédé selon la revendication 5, dans lequel ledit facteur hématopoïétique est G-CSF.

8. Procédé selon la revendication 7, dans lequel le G-CSF est G-CSF humain naturel ou est obtenu comme un produit d'expression de cellule hôte procaryote ou eucaryote.

9. Procédé selon la revendication 7, dans lequel le G-CSF est G-CSF modifié chimiquement.

10. Procédé selon la revendication 9, dans lequel le G-CSF modifié chimiquement est G-CSF pégylé.

11. Procédé selon la revendication 1, dans lequel ladite protéine est rhG-CSF dérivé de E. Coli, dans lequel ladite vésicule liposome est DOPG, et dans lequel ladite composition a un rapport de 50 : 1 de DOPG : rhG-CSF, a un pH de 4,5 et contient de l'acétate de sodium 10 mM.
